# EUROPEAN PATENT SPECIFICATION

(11) **EP 0 992 506 B1**
(45) Date of publication and mention of the grant of the patent: **06.08.2003**
(21) Application number: 99119768.2
(22) Date of filing: 06.10.1999
(51) Int. Cl.: C07F 9/17, C07H 21/00

(54) **Process for the sulfurisation of phosphorus-containing compounds**
Verfahren zur Sulfurierung von Phosphor-enthaltenden Verbindungen
Procédé pour la sulfurisation de composés contenant du phosphore

(30) Priority: 08.10.1998 US 168447
(43) Date of publication of application: 12.04.2000
(73) Proprietor: Novartis AG, 4056 Basel (CH); Novartis Pharma GmbH, 1230 Wien (AT)
(72) Inventor: Martin, Pierre, 4310 Rheinfelden (CH); Natt, Francois Jean Charles, 4147 Aesch (CH)
(74) Representative: Grubb, Philip William

(56) References cited:
- WO-A-97/41130
- WO-A-98/54198
- GOERDELER J. ET AL.: "Herstellung von Acylheterocumulenen aus fünfgliedrigen Ringen mit Hilfe von Phosphinen" CHEMISCHE BERICHTE., vol. 107, no. 2, 1974, pages 502-507, XP002173760 VERLAG CHEMIE GMBH. WEINHEIM., DE ISSN: 0009-2940

## Description

Phosphorothioate analogues of the phosphate moiety in compounds are of great interest in nucleic acid research , protein research, etc. For example, phosphorothioate-containing antisense oligonucleotides have been used in vitro and in vivo as inhibitors of gene expression.
Introduction of phosphorothioate moieties into oligonucleotides, assembled by solid-phase synthesis, can be achieved using either an H-phosphonate approach or a phosphoramidite approach. The H-phosphonate approach involves a single sulfur transfer step, carried out after the desired sequence has been assembled, to convert all of the internucleotide linkages to phosphorothioates. Alternatively, the phosphoramidite approach features a choice at each synthetic cycle: a standard oxidation provides the normal phosphodiester internucleotide linkage, whereas a sulfurization step introduces a phosphorothioate at the specific position in the sequence. An advantage of using phosphoroamidite chemistry, therefore, is the capability to control the state of each linkage in a site specific manner.
The success of the phosphoramidite approach is dependent on the availability of efficient, good soluble sulfurization reagents that are compatible with automated DNA synthesis.
A number of reagents have been designed and tested in recent years (WO 97/41130) but none of them is able to fulfil all the requirements for an ideal sulfurization reagent: excellent yields, good solubility, stable solutions, short reaction times, no formation of P=O units, no further reagents necessary, no side reactions with other parts of the molecule, odorless itself and odorless reaction products, capable of regeneration, and easily available.
Goerdeler et al. (Chem Ber. 107 (1974), 502) reacted 5-amino-1,2,4-dithiazol-3-thione compounds with triphenylphosphine in order to prepare acylheterocumulenes. However, neither yield nor structure of Triphenylphosphine sulfide was characterized.

The present invention provides a process for the sulfurization of phosphorus-containing compounds. This process involves contacting the compound to be sulfurized with a sulfur transfer reagent of formula (I) 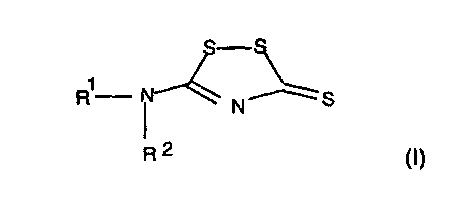 wherein
- R¹: is aryl which can be substituted by halogene, (C₁ - C₆) alkyl, (C₁ - C₆) alkoxy
- R²: is (C₁ - C₆) alkyl, which could also form a cycle together with R¹.

Preferred are sulfur transfer reagents of formula (I) wherein
- R¹: is phenyl, tolyl, xylyl, naphthyl, 4-chlorophenyl, anisyl
- R²: is methyl, ethyl, propyl, isopropyl
also preferred are sulfur transfer reagents of formula (II) 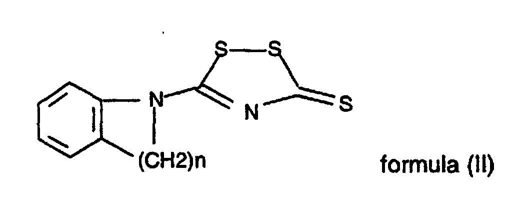 wherein n is 2 or 3.

The method of the present invention typically involves contacting a sulfur transfer reagent of formula (I) with a phosphorus-containing compound in solution or on a solid support, in a solvent or a mixture of solvents. The phosphorus in the phosphorus-containing compound is typically a trivalent phosphorus. Upon sulfurization it becomes a pentavalent phosphorus.
The trivalent phosphorus can be a phosphite, phosphonite, phosphonamidite, phosphine or any other phosphorus (III) derivative as part of the synthesis of DNA, RNA, phosphoropeptides, phosphonopeptides, phosphorylated nucleoside sugars or oligosaccharides.
Preferably the phosphorus containing compound is a compound of formula (III) 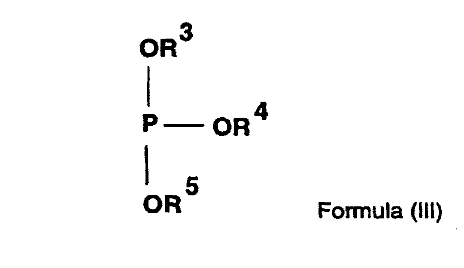 wherein R³ and R⁴ are a nucleoside or an oligonucleotide
and R⁵ is a protective group, especially R⁵ is -CH₂CH₂CN, -CH₂-CH=CH-CH₂CN, -CH₂CH₂ -4-nitrophenyl, -CH₂CH=CH_{2..}

For oligonucleotides the sulfur transfer reagents of formula (I) or (II) do not modify the nucleosidic residues, thereby preserving the genetic identity of the macromolecule. Thus the reagents of formula (I) or (II) and the process of the present invention can be reliably used in the automated synthesis of desired compounds. For example the process is very useful for the automated synthesis up to gram scales of oligonucleotides, including both oligodeoxyribonucleotides and oligoribonucleotides of length from 4 to 50 bases containing the phosphorothioate substitution at either a single site or at all positions.
Typically an amount of 1 - 30 molar equivalents, more preferably 2 - 10 molar equivalents) of the sulfur transfer reagent of formula (I) or (II) is used relative to the amount of the trivalent phosphorus groups in the phosphorus-containing compound. The reaction is typically carried out under an inert atmosphere, such as argon, although this is not required.

The sulfurization reaction occurs in a solvent. The solvent can be a hydrocarbon solvent, ethereal solvent, nitrile solvent, chlorinated solvent, heterocyclic solvent etc.. Specific examples of suitable solvents include pyridine, N,N-dimethylformamide (DMF), tetrahydrofuran (THF), acetonitrile, methylenechloride. Preferably acetonitrile is used.

The reaction can be carried out at room temperature, but also within a range of about 0 - 50°C and preferably 10 - 50°C. Typically the conversion to the thioated compound is greater than 95%, mostly greater than about 99%.

A great advantage of the process of the present invention is that the compounds of formula (I) or (II) could be easily recovered from the reaction solution obtained from the automated synthesizer by reacting these solutions with sulfur or sulfur transfer reagents.
In contrast such recycling is not possible with the compounds described in WO 97/41130. Furthermore the sulfurization reaction described therein (see page 15 of WO 97/41130) lead to the formation of carbonoxysulfide (COS) which is an easily flammable gas with an horrible odour. This formation of COS causes practical and safety problems.

The compounds of formula (I) and (II) could be prepared by the reaction of the corresponding thioureas with CS₂ and subsequent oxidation (DT 2404477 A1). The following examples shall illustrate the invention but not restrict it.

### Examples

Preparation of the thioureas 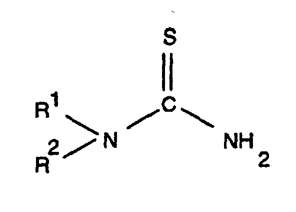 N-Ethylphenyl-thiourea (R¹ = phenyl, R² = ethyl)

A solution of 35,0 g (0,288 mol) ethylaniline and 46,8 g (0,577 mol) NaSCN in 220 ml toluene is heated to 50°C and a mixture 31 ml trifluoroacetic acid and 45 ml toluene is added during 3,5 h. The reaction mixture is refluxed for 2,5 h. After cooling the mixture is washed with water, dried and evaporated. The residue is treated with ether. After filtration a beige powder is obtained, mp. 104 to 105 °C.

Analogue are prepared:

| R¹ | R² | MP (°C) | MS (M⁺) |
|---|---|---|---|
| 2,3-dimethylphenyl | ethyl | 93-94 | 208 |
| 4-methoxyphenyl | methyl | 126 | 196 |
| 1-naphthyl | ethyl | 124-125 | 230 |
| phenyl | methyl | 105-106 | 166 |
| 4-chlorophenyl | methyl | 125 | 200/202 |
| indoline | | 156 | 178 |
| tetrahydrochinoline | | 134-135 | 192 |

### Preparation of compounds of formula (I)

### Method A)

### Preparation of N-ethyl,N-phenyl-5-amino-3H-1,2,4-dithiazol-3-thione (R¹ = Phenyl, R² = ethyl)

To a stirred mixture of 29,52 g (0,164 mmol) N-ethyl-phenyl-thiourea, 14,9 g CS₂ and 400 ml THF 8,17 g (0,34 mol) NaH is added in portions. After the production of hydrogen has been ceased the reaction mixture is heated for 2,5 h. After cooling the precipitate is filtered, dissolved in 300 ml H₂O and a solution of 41,56 g I₂ and 81,55 g Kl in 500 ml H₂O is added dropwise. The formed precipitate is filtered off and washed with hexane. Obtained are yellow crystals, Mp 141°C.

### Method B)

To a solution of 11,1 mmol thiourea in 20 ml DMSO 0,87 ml CS₂ and 1,56 g powdered KOH are added. After stirring for 24 h the reactionmixture is poured into water and extracted with EtOAc. The extract is washed with water, dried (Na₂SO₄) and evaporated. The residue is chromatographed (SiO₂, Hexan:EtOAc 6:1).

Analogue (method A or B) are prepared:

| R¹ | R² | MS (M⁺) |
|---|---|---|
| 2,3-dimethylphenyl | ethyl | 282 |
| 4-methoxyphenyl | methyl | 270 |
| naphthyl | ethyl | 304 |
| phenyl | methyl | 240 |
| 4-chlorophenyl | methyl | 274/276 |
| indoline | | 252 |
| tetrahydrochinoline | | 266 |

### Sulfurization reaction

### Synthesis of phosphorothioate containing oligonucleotides

For 1 µmole scale syntheses, a Perseptive Expedite MOSS synthesizer is used. The syntheses are performed on Polystyrene Primer support (Pharmacia) loaded with the 3' end residue. Solutions at 0.05M in acetonitrile of β-cyanoethyl deoxyribonucleosides phosphoramidites (Perseptive) or β-cyanoethyl 2'-methoxyethylribonucleosides phosphoramidites (P. Martin, Helvetica Chimica Acta, 78 (1995), 486 - 504) are used. In the coupling step, phosphoramidites are activated by benzimidazolium triflate (0.2M in acetonitrile; R. Noyori, J.Org. Chem. 61, 1996, 7996 - 7997). Non sulfurized phosphodiesters are oxidized by anhydrous t-butyl hydroperoxide (0.5M in toluene; this solution is obtained by diluting the toluene solution of t-butyl hydroperoxide from Lipomed). Capping and washing steps are carried out with standard reagents and solvents.

Typical cycles as well as the sulfurization conditions are shown in Table 1. The sulfur transfer reagent was used at a 0.1M concentration in acetonitrile. The volume of sulfurization reagent was 1.28 ml (including prime and purge volumes). Total contact time was 2 minutes.

Upon completion of solid-phase steps, the oligonucleotides (5' trityl-off) are cleaved from the support and deprotected with 30% ammonium hydroxide (2h at room temperature for polypyrimidines and at 80°C for mixed sequences), desalted on NAP-10 columns (Pharmacia) and analyzed by capillary gel electrophoresis and Maldi-Tof MS (see Table 2).

Alternatively, the same process is applicable to trityl-on oligonucleotides suited for RP-HPLC purification.

**Table 1:**

| Synthetic conditions | | | |
|---|---|---|---|
| Step | Reagent/Solvent | Function | Time in sec. per cycle (repeat) |
| 1 | MeCN | Wash | 20 (2x) |
| 2 | 3% CCl₃COOH/CH₂Cl₂ | Detritylation | 20+40 |
| 3 | MeCN | Wash | 20 (2x) |
| 4 | Nucleotide/tetrazole/MeCN | Coupling | 180 |
| 5 | MeCN | Wash | 20 (2x) |
| 6 | PM-5468 0.1 M in acetonitrile or 0.5M tBuOOH in DCM | Sulfurization/Oxidat ion | 20 (2x) |
| 7 | MeCN | Wash | 20 (2x) |
| 8 | Ac₂O/lutidine/NMI/THF | Capping | 10+20 |
| 9 | MeCN | Wash | 20 (2x) |

**Table 2:**

| sequences and results (N: DNA; n: 2'-methoxyethyl RNA; s: phosphorothioate) | | |
|---|---|---|
| Sequence | MW_{calc.} | MW_{meas.} |
| TsTT TTT TT | 2387.7 | 2387.7 |
| TTT TsTT TT | 2387.7 | 2386.5 |
| tstt ttt tt | 2980.4 | 2984.8 |
| ttt tstt tt | 2980.4 | 2985.6 |
| AsAsTs CsCsTs CsCsCs CsCsAs GsTsTs CsAsCs CsC | 6223.2 | 6222.8 |

### C) Solid Phase Synthesis of a 20-mer full phosphorothiate oligodeoxyribonucleotide

Syntheses are performed on a Pharmacia OligoPilot II using 6.3ml (150-200 µmole scale) or 24ml (500 µmole scale) columns. Same reagents are used as in example B except for the support (Primer 30 HL loaded with the first 3' deoxyribonucleoside via a succinyl linker) and the phosphoramidites are dissolved as a 0.2M solution in acetonitrile.
Synthesis scale is based on weight and loading of the support. Excess and crude yields are calculated from the synthesis scale.
1.5 equivalent DNA phosphoramidite and the same excess of Benzimidazolium Triflate are simultaneously added to the column and recycled for 3 minutes. The synthetic conditions are shown in the table 3. The specific conditions for the sulfurization as well as the results are shown in table 4. Crude material contained between 67 and 72% of full length material.

**Table 3:**

| Synthetic conditions on the OligoPilot II (Pharmacia) | | | |
|---|---|---|---|
| Step | Reagent/Solvent | Function | Volume or equiv. |
| 1 | MeCN | Wash | 6 CV |
| 2 | 3% CCl₃COOH/CH₂Cl₂ | Detritylation | 6 CV |
| 3 | MeCN | Wash | 6 CV |
| 4 | Nucleotide/tetrazole/MeCN | Coupling | 1.5 equiv |
| 5 | MeCN | Wash | 6 CV |
| 6 | STR | Sulfurization | See table 4 |
| 7 | MeCN | Wash | 6 CV |
| 8 | Ac₂O/lutidine/NMI/THF | Capping | 0.5 CV; 0.5 min. |
| 9 | MeCN | Wash | 6 CV |

**Table 4:**

| Table 4: synthesis of the 20-mer phosphorothioate **1** 5'- AsAsTs CsCsTs CsCsCs CsCsAs GsTsTs CsAsCs CsC and the 20-mer chimeric 2'-methoxyethyl RNA/DNA phosphorothioate **2** 5'-GsTsGs AsGsGs CsCsCs TsGsts tsgsas gststs gsg (n is 2'-methoxyethyl residue, N is DNA residue, s is phosphorothioate) | | | | | | |
|---|---|---|---|---|---|---|
| OLIGO | STR | Solvent | Conc.(M) | Excess | Crude yield | P=O (³¹P-NMR) |
| **1** | Van | CH3CN/ | 0.5 | 8 | 63.6% | <0.5% |
| | Boom | Picoline | | | | |
| **1** | Van | CH3CN/ | 0.5 | 4 | 60.4% | <0.5% |
| | Boom | Picoline | | | | |
| **1** | PM-5468 | CH3CN | 0.1 | 8 | 66.5% | <0.5% |
| **1** | PM-5468 | CH3CN | 0.1 | 4 | 68,8% | <0.5% |
| **1** | PM-5468 | THF | 0.2 | 4 | 68% | <0.5% |
| **2** | PM-5468 | CH3CN | 0.1 | 4 | 82% | <0.5% |
| Abbreviations: | | | | | | |
| STR: Sulfur Transfer reagent | | | | | | |
| PM-5468: Compound of formula (I) wherein R¹ is Phenyl and R ² is Ethyl | | | | | | |
| Van Boom: Phenyl Acetyl DiSulfide also named as PADS. | | | | | | |
| BT: Benzimidazolium Triflate | | | | | | |

Recyling of the reagent from the solutions obtained after the sulfurization step at the synthesizer

### Method A)

To the solutions collected after the sufurization step from the synthesizer, sulfur or sulfur dissolved in CS₂ is added. After 2 days the solutions are filtered and evaporated. The residue is dissolved in EtOAc and filtert over a short SiO₂-column. After evaporation and washing of the residue with hexane pure compounds of formula (I) could be obtained.

### Method B)

The solutions collected after the sufurization step from the synthesizer are evaporated. To the solution of the residue in acetone sulfur is added, the mixture is refluxed for 2 h and evaporated. The residue is treated with acetonitrile, filtered, evaporated and chromatographed. Pure compounds of formula (I) are obtained.

Example: Comparison of sulfurization efficiencies of three sulfur transfer reagents

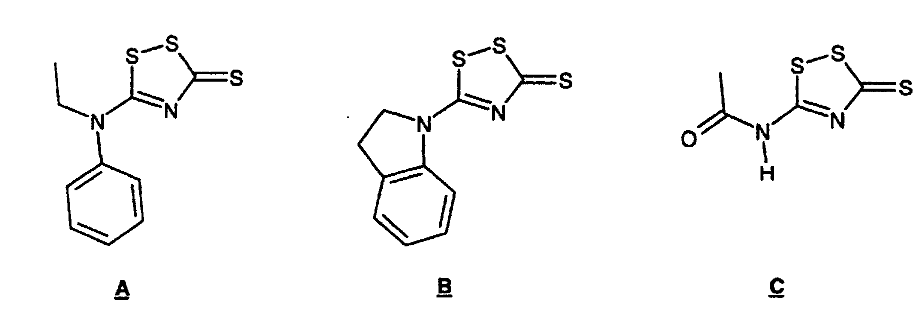

The purpose of the experiments is the head to head comparison of the three mentionned products (namely, **A**, **B**,**C**), the third being described in Patent WO98/54198. In this reference, the compound **C** is used for the preparation of phosphorothioate oligonucleotides with a 0.02M (12 equiv.)solution in Pyridine/acetonitrile (1:9). As the use of pyridine and the high excess required for this experiment are considered to be drawbacks of a STR, we compare the three reagents with "ideal" or satisfactory conditions. Compound **C** is not soluble at 0.1 M in acetonitrile nor THF which are the recommended conditions for compounds **A** and **B**.

The test sequence is a polythymidine (12-mer) where in the middle of the sequence, the residue to be sulfurized is a 2'-O-methoxyethyl thymidine. The sulfurization is carried out with 0.02M (2 equiv.) in Acetonitrile for 2 min. When specified the support is subsequently exposed to oxidation for further conversion of phosphite species The crude solutions are analysed by Maldi-Tof MS, CGE and IE-HPLC, results are summarized in table 5.

**Table 5**

| analyses of crude oligonucleotide TTT TTts TTT TTT | | | | | |
|---|---|---|---|---|---|
| entry | STR | Crude yield (OD) | % CGE | % P=O (IE-HPLC)^{b} | Maldi-Tof MS (MWcalc.=3678.6) |
| 1 | **A** | 97 | 98.2 | n.d. | 3676.9 |
| 2 | **B** | 91 | 97.5 | n.d. | 3677.2 |
| 3 | **C** | 88 | 73.3 | 10^{c} | 3675.7; 3280.6; 1896.5; 1757.7 |
| 4 | **A**^{a} | 95 | 98.8 | n.d. | 3676.6 |
| 5 | **C**^{a} | 90 | 97.8 | 17.7 | 3676.6 |

| | | | | | |
|---|---|---|---|---|---|
| ^{a} sulfurization is followed by a oxidation with tBuOOH as already described | | | | | |
| ^{b} not detected | | | | | |
| ^{c} 7.8% of cleavage products | | | | | |

### Discussion:

Both STR **A** and **B** sulfurize the 2'-O-methoxyethyl residue in a satisfactory yield.
Under these conditions, it appears that STR **C** does not sulfurize completely the internucleotidic bridge which leads a) to a partial cleavage of the remaining phosphite during detritylation following the sulfurization and b) to the oxidation of the non cleaved phosphite in the next chain elongation cycle.
When the sulfurization is followed by an additional oxidation before further chain elongation (entry 5), no cleavage product is observed, but the IE-HPLC analysis shows a much higher P=O content as the corresponding experiment with STR **A**.

## Claims

1. A process for the sulfurization of a compound of formula (III) 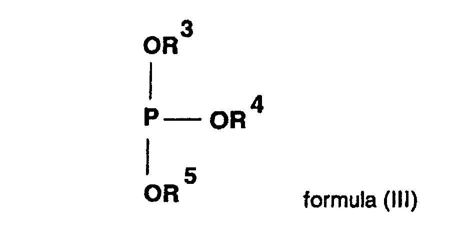 wherein each of R³ and R⁴ is a nucleoside or an oligonucleotide
and R⁵ is a protective group.
which comprises contacting the compound of formula (III) with a sulfur transfer reagent of formula (I) 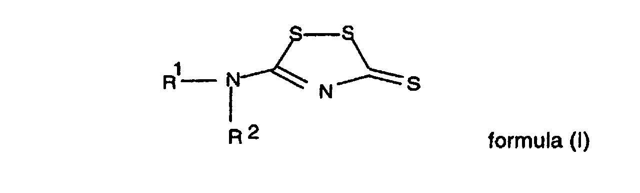 wherein
R¹ is aryl which can be substituted by halogene, (C₁ - C₆) alkyl or (C₁ - C₆) alkoxy, and
R² is (C₁ - C₆) alkyl, which could also form a cyclic ring together with R¹.

2. A process according to claim1 wherein
R¹ is phenyl, tolyl, xylyl, naphthyl, 4-chlorophenyl or anisyl, and
R² is methyl, ethyl, propyl or isopropyl.

3. A process according to claim 1 wherein the sulfur transfer reagent is a compound of formula (II) 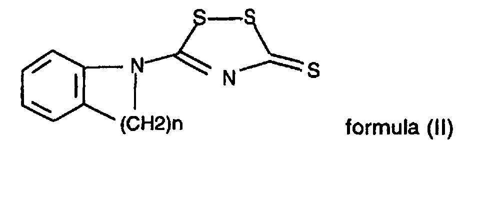 wherein n is 2 or 3.

4. A process according to claim 1, 2 or 3 wherein R⁵ is -CH₂CH₂CN, -CH₂-CH=CH-CH₂CN, -CH₂CH₂ -4-nitrophenyl or -CH₂CH=CH₂.

5. A process according to any of the previous claims wherein the sulfur transfer reagent is used in an amount of 1 - 30 molar equivalents, preferred 2 - 10 molar equivalents relative to the amount of the phosphorus compound.

6. A process according to any of the previous claims wherein the reaction is carried out in the presence of pyridine, DMF, THF, acetonitrile or methylenechloride, preferably acetonitrile as a solvent.

7. Use of compounds of formula (I) for the sulfurization of phosphorus compounds of formula (III).

8. Use of compounds of formula (I) for the sulfurization of nucleotides or oligonucleotides.

9. Use of compounds of the formula (II) for the sulfurization of nucleotides or oligonucleotides.

## Patentansprüche

1. Verfahren zum Sulfurieren einer Verbindung der Formel (III) 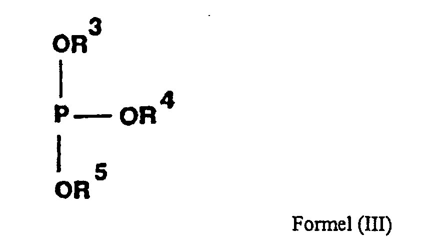 wobei R³ und R⁴ jeweils ein Nucleosid oder ein Oligonucleotid und R⁵ eine Schutzgruppe ist, das
das Inkontaktbringen der Verbindung der Formel (III) mit einem Schwefeltransferreagenz der Formel (I) umfasst 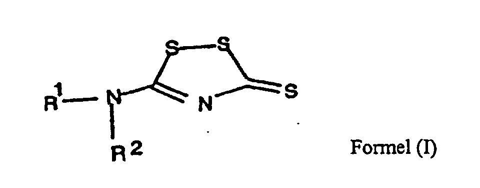 wobei
R¹ für Aryl steht, das mit einem Halogen, (C₁-C₆) Alkyl oder (C₁-C₆) Alkoxy substituiert sein kann, und
R² für (C₁-C₆) Alkyl steht, das auch einen cyclischen Ring zusammen mit R¹ bilden könnte.

2. Verfahren nach Anspruch 1, bei dem
R¹ für Phenyl, Tolyl, Xylyl, Naphthyl, 4-Chlorphenyl oder Anisyl steht, und
R² für Methyl, Ethyl, Propyl oder Isopropyl steht.

3. Verfahren nach Anspruch 1, bei dem das Schwefeltransferreagenz eine Verbindung der Formel (II) ist 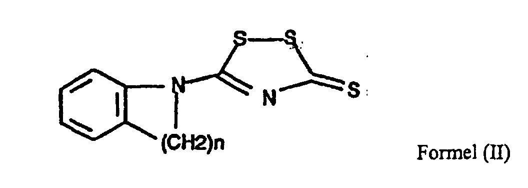 wobei n für 2 oder 3 steht.

4. Verfahren nach Anspruch 1, 2 oder 3, bei dem R⁵ für -CH₂CH₂CN, -CH₂-CH=CH-CH₂CN, -CH₂CH₂-4-Nitrophenyl oder -CH₂CH=CH₂ steht.

5. Verfahren nach einem der vorstehenden Ansprüche, bei dem das Schwefeltransferreagenz in einer Menge von 1 bis 30 molaren Äquivalenten, bevorzugt 2 bis 10 molaren Äquivalenten, relativ zu der Menge der Phosphorverbindung verwendet wird.

6. Verfahren nach einem der vorstehenden Ansprüche, bei dem die Reaktion in Anwesenheit von Pyridin, DMF, THF, Acetonitril oder Methylenchlorid, bevorzugt Acetonitril, als ein Lösemittel ausgeführt wird.

7. Verwendung von Verbindungen der Formel (I) für die Sulfurierung von Phosphorverbindungen der Formel (III).

8. Verwendung von Verbindungen der Formel (I) für die Sulfurierung von Nucleotiden oder Oligonucleotiden.

9. Verwendung von Verbindungen der Formel (II) für die Sulfurierung von Nucleotiden oder Oligonucleotiden.

## Revendications

1. Procédé de sulfuration d'un composé de formule (III) 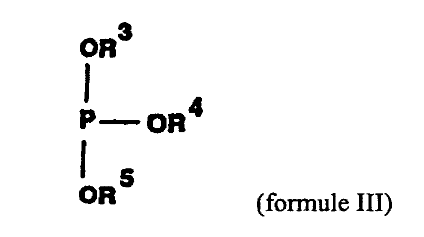 dans laquelle chacun des radicaux R³ et R⁴ est un nucléoside ou un oligonucléotide,
et R⁵ est un groupe protecteur,
lequel comprend la mise en contact du composé de formule (III) avec un réactif de transfert de soufre de formule (I) 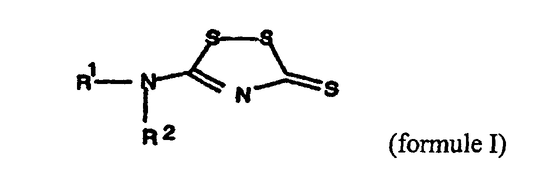 dans laquelle
R¹ est un aryle qui peut être substitué par un halogène, un alkyle en C₁-C₆ ou un alcoxy en C₁-C₆, et
R² est un alkyle en C₁-C₆, qui peut aussi former un groupe cyclique avec R¹.

2. Procédé selon la revendication 1, dans lequel
R¹ est un groupe phényle, tolyle, xylyle, naphtyle, 4-chlorophényle ou anisyle, et
R² est un groupe méthyle, éthyle, propyle ou isopropyle.

3. Procédé selon la revendication 1, dans lequel le réactif de transfert de soufre est un composé de formule (II) 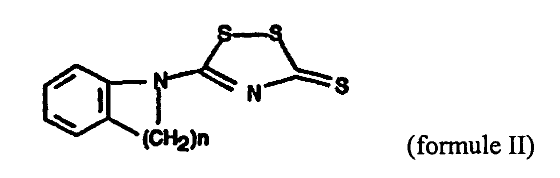 dans laquelle n vaut 2 ou 3.

4. Procédé selon la revendication 1, 2 ou 3, dans lequel R⁵ est -CH₂CH₂CN, -CH₂-CH=CH-CH₂CN, -CH₂CH₂-4-nitrophényle ou -CH₂CH=CH₂.

5. Procédé selon l'une quelconque des revendications précédentes, dans lequel le réactif de transfert de soufre est utilisé dans une quantité comprise entre 1 et 30 équivalents molaires, de préférence entre 2 et 10 équivalents molaires, par rapport à la quantité du composé de phosphore.

6. Procédé selon l'une quelconque des revendications précédentes, dans lequel la réaction est réalisée en présence de pyridine, de DMF, de THF, d'acétonitrile ou de chlorure de méthylène, de préférence d'acétonitrile en tant que solvant.

7. Utilisation de composés de formule (I) pour la sulfuration des composés phosphorés de formule (III).

8. Utilisation de composés de formule (I) pour la sulfuration de nucléotides ou d'oligonucléotides.

9. Utilisation de composés de formule (II) pour la sulfuration de nucléotides ou d'oligonucléotides.
